# EUROPEAN PATENT SPECIFICATION

(11) **EP 1 314 000 B1**
(45) Date of publication and mention of the grant of the patent: **13.05.2009**
(21) Application number: 01960205.1
(22) Date of filing: 24.08.2001
(51) Int. Cl.: G01B 11/24, A61B 1/05

(54) **METHOD AND APPARATUS FOR THREE-DIMENSIONAL OPTICAL SCANNING OF INTERIOR SURFACES**
VERFAHREN UND APPARAT FÜR OPTISCHE DREIDIMENSIONAL-ABTASTUNG INTERNER OBERFLÄCHEN
PROCEDE ET APPAREIL DESTINE AU BALAYAGE OPTIQUE EN TROIS DIMENSIONS DE SURFACES INTERIEURES

(30) Priority: 25.08.2000 DK 200001258; 31.10.2000 US 244561 P
(43) Date of publication of application: 28.05.2003
(73) Proprietor: 3Shape APS, 1060 Copenhagen K (DK)
(72) Inventor: FISKER, Rune, DK-2830 Virum (DK); CLAUSEN, Tais, DK-2100 Kobenhavn (DK); DEICHMANN, Nikolaj, DK-2100 Copenhagen Ø (DK); VASILJEV BARTHE, Christophe, DK-2200 Kobenhavn N (DK)
(74) Representative: HOEIBERG A/S
(86) International application number: PCT/DK2001/000561
(87) International publication number: WO 2002/016867

(56) References cited:
- EP-A- 0 352 952
- WO-A-97/32182
- DE-A- 4 102 614
- DE-A- 19 803 679
- US-A- 5 172 685

## Description

### 1. Technical field

The invention relates to the creation of high precision three-dimensional replicas of real objects. The invention specifically concerns the three-dimensional scanning of interior surfaces or cavities of limited dimensions or with restricted accessibility. Furthermore, the invention relates to a specific embodiment for scanning the human ear and ear canal.

### 2. Prior art

Systems for three-dimensional optical scanning are well known in the prior art. They typically comprise one or more light sources projecting a structured light pattern on the object to be scanned, one or more cameras and data processing equipment to convert the recorded image co-ordinates to three dimensional co-ordinates using state of the art software. Usually, only a part of the object is scanned in a single scan. To create a full scan the object, camera and light source need to be move relative to each other.

Precision is of utmost importance in many applications, e.g. when the scan data is used to model an object, which must fit precisely into another part. Such applications are e.g. devices for the ear canal such as hearing aids, dental implants and other prostheses for the body. For hearing aid shells sub-millimetre precision is required or the shell will cause irritation, acoustic feedback and possibly infection to the epidermis of the ear canal. For dental implants the precision requirement is even greater, since a human being can detect differences less than 1/10 of a millimetre when biting. Therefore systematic or random errors in the calibration and/or performance of scanners for these uses can be fatal. This has hitherto limited the use of scanning in the modelling of such implants and shells.

WO 96/10204 (Fright et al.) discloses a handheld 3-D scanner, which uses a position sensor to determine the relative position of the scanner with respect to the object to be scanned. The disclosed scanner is not adapted to scan interior surfaces due to its size and due to the fact that the light sources point towards the centre of in which the object to be scanned must be placed. This scanner thus suffers from the same occlusion effects as non-portable scanners. The patent application is the basis for the Polhemus FastTrack handheld scanner.

US 6,263,234 (Leica Microsystems Heidelberg GmbH) discloses a confocal surface-measuring device, which is adapted to emit a ray of light and partly move this ray in relation to the axis of the device to perform a scan of an area below the device. The invention dos not disclose how the light reflected from the object is directed to the detector, which makes it very doubtful whether the construction is able to work in practice. However the scanner only rotates the light deflecting means and not the construction related to the detector, which only makes it able to scan a very limited part of the circumference.

US 5,895,927 (USA Secretary of the Air Force) discloses a non-contact interior cross-section profiler for measuring internal dimensions of pipes, tubes etc. The profiler generates a disc of light using a cone shaped mirror which is reflected from the interior surface of the pipes, tubes etc. to a camera. It is however doubtful whether the profiler can measure the dimensions precisely, since small errors in the orientation of the profiler along the axis of the pipe or tube will cause the disc to be distorted. The profiler is only meant to measure internal dimensions and not to produce 3-D real world co-ordinates.

US 5,784,098 (Olympus Optical Co., Ltd.) discloses an endoscope which is capable of performing 3D scanning of small areas in the interior of a human being. The scanner works by projecting a pattern consisting of several sheets of light from the tip of the endoscope onto the surface in front of the tip. 2-D images are taken by a camera connected via light guides to the tip of the endoscope. Due to this construction, the angle at which light is reflected from the surface towards the camera is very small and not optimal for precision scanning. Furthermore, the scanner is only adapted to map small areas. Additionally the design is very likely to give very distorted images, which makes high precision scanning infeasible.

WO 01/22030 (Mycrona Gesellschaft für Innovative Messtechnik GmbH) discloses a device for measurement of poorly accessible hollows such as the inside of a bore. The device is capable of measuring the internal diameter of the hollow. However the scanner is only able of perform scans at maximal 180° of the circumference, since it only applies one or two static coplanar mirrors.

US 5,056,204 (Ascom Audiosys AG) concerns a method for milling of hearing aids whereby the internal contours of the ear canal are allegedly recorded by a laser apparatus located outside the ear of the patient. The disclosure contains no means to direct the laser light into the ear canal. Due to occlusion effects only part of the ear canal can be scanned according to the disclosed method. Furthermore, the disclosure fails to take regard to the fact that the individual is likely to move while the images are recorded.

WO 00/34739 (Fagan et al.) concerns a method for manufacturing hearing aid shells involving the use of a specially adapted ultrasonic scanner head to safely measure the contours of the ear canal without contact with the surface being measured. The recording of the data in the ear canal is made possible by filling the canal with a liquid and inserting the ultrasonic scanner. The scan data are processed by a computer and the data are used with a rapid prototyping set-up such as stereo lithography, selective laser sintering, laminate object modelling, inkjet modelling, fused depositing modelling, 3DP or any other system that produces real models from computer mathematical models to manufacture the hearing aid shell. One disadvantage of the system is the need for filling the patient's ear completely with water. This can be extremely annoying to patients and they may experience symptoms of nausea during and after such treatment. Furthermore it is doubtful whether it is really possible to determine the three dimensional co-ordinates of the surface of the ear canal with the required precision using an ultra sonic scanner. An ultrasonic sound signal emitted does not travel in just one direction in the liquid in the ear canal. As the ultrasonic waves are reflected by the surface of the ear canal they travel in many different directions. Thus the transducers of the ultrasonic scanner will detect a number of signals of varying size with different delays after the emission of one sound signal. It must be extremely difficult for the underlying software to determine which of the signals to use in the determination of the coordinates. The disclosure provides no information on how to perform this operation.

EP 0 516 808 (Tøpholm & Westermann Aps) concerns a method for computer assisted manufacture of otoplastic individually fitted to the contours of the ear canal.

According to the described method a digital representation of the internal contours of the ear canal is used for milling or 3D printing of a hearing aid shell. A computer is used to optimise the location of the components of the hearing aid and the thickness of the walls of the shell. The disclosure does not suggest the solution of scanning the internal contours of the ear canal using an optical scanner.

### 3. Summary of the invention

According to a first aspect the invention relates to a scanner for three-dimensional scanning of interior surfaces, comprising
at least one light source adapted to create and project structured light producing a pattern on the interior surface of an object,
at least one camera, adapted to record 2-D images of the pattern,
data processing means adapted to convert 2-D image information into 3-D real world co-ordinates,
at least one position sensor adapted to provide the relative position and orientation of the scanner during successive scans,
the point of emission of light as well as the point of accumulation of reflected light for the camera being located on a probe having an axis,
the at least one light source and the at least one camera being adapted to perform a scan 360° around the axis, and
the probe being adapted to be entered into a cavity.

The probe of the scanner may be either rigid of flexible

Compared to prior art scanners this scanner has the advantage that it is able to cover the whole circumference without moving the scanner thus being able to scan the whole inner surface of an object. With this layout it is possible to scan interior surfaces such as the ear canal, tubes, pipes and bores with non-contact scanning and obtain high precision scan data of the whole interior surface of the object.

Furthermore, the dimensions of the scanner can be very small thus allowing scanning and 3D mapping of interior surfaces with small cross section, which are inaccessible to prior art scanners.

According to an especially preferred embodiment the scanner is equipped with a position sensor, which allows the relative position and orientation of the scanner and the object to be determined for successive scans. This greatly facilitates the combination of data from successive scans and makes it possible to combine these with much higher precision irrespective of the position and orientation of the scanner during scanning.

The compact layout of the scanner allows for easy scanning of interior surfaces of objects of extremely small size. The ease of operation of the scanners according to the invention means that practitioners without experience in scanning can easily perform the scanning operations, which is required especially in the case of scanning of body cavities and scanning for archaeological purposes.

According to a further aspect the invention relates to a method for scanning interior surfaces comprising the steps of
i) entering a probe shaped scanner having an axis into a cavity,
ii) creating and projecting structured light from a first point on the probe producing a pattern on an interior surface of an object, and at a second point of the probe, recording 2D images of the pattern reflected from the interior surface, thereby performing a scan 360° around the axis of the probe,
iii) determining 2D co-ordinates of the images of the pattern,
iv) determining the relative position and orientation of the scanner during successive sans with at least one position sensor,
v) combining a series of images to obtain 3D real world co-ordinates of the interior surface.

The method allows for easy scanning of interior surfaces of objects which cannot be scanned with high precision using prior art scanning methods.

Preferably the method is carried out with a scanner according to the invention.

According to a preferred embodiment the method for scanning further comprises calibration of the scanner by:
i) scanning a three dimensional calibration object having at least one plane of symmetry and whereby at least part of at least one 3D object feature curve of each symmetric part is a continuous curve,
ii) determining image feature co-ordinates being representations of at least one pair of 3D object feature curves for each of a discrete number of values of an angle or rotation and/or translation, a pair consisting of one 3D object feature curve in each symmetric part of the calibration object,
iii) changing the calibration parameters to fit the calibration object.

According to a still further aspect the invention relates to a method for 3D modelling and production comprising obtaining 3D real world co-ordinates of an interior surface of a cavity provided using the method according to the invention, and creating a piece adapted to fit into the cavity.

Thereby, the steps for manufacturing the piece are reduced to the absolute minimum and an especially perfect fit of the piece can be obtained. The interior surface may be scanned a number of times, such as under different conditions affecting the geometry of the interior surface. Thereby the variations in the dimensions of the interior surface can be recorded. This is very cumbersome using the prior art techniques.

Once the data are recorded the piece may be manufactured using any automatic manufacturing technique such as milling. More preferably the modelling technique comprises 3-dimensional printing, stereo lithography, selective laser sintering, laminated object modelling, inkjet modelling, fused deposition modelling, nano-printing. A common feature of these techniques is that only the required amount of material is used and that it is easier to produce complex models such as devices for the ear and/or ear canal and/or dental implants.

The devices for the ear may comprise a hearing aid, a mobile phone, a loud speaker, a microphone, communication devices, a tinnitus masker or a tinnitus masking device such as the ones described in US 5,325,872 and WO 91/17638. Figure 13 shows a scan of the interior surface of an ear and an ear canal 1301.

### 4. Brief description of the drawings

Figure 1 illustrates an embodiment of the interior surface scanner according to the invention.
Figure 2 shows a cross section of an embodiment of the interior surface scanner according to the invention.
Figure 3 illustrates another embodiment of the interior surface scanner with a mirror in front of the camera.
Figure 4 shows a cross section of another embodiment of the interior surface scanner with a mirror in front of the camera.
Figure 5 shows how a structured light pattern is projected onto the interior surface. In this case the pattern is a single cone. This pattern is then reflected from the surface into the camera.
Figure 6 illustrates an example of the use of mirrors and/or prisms. A structured light pattern is reflected in a mirror before being projected onto the interior surface. In this case the pattern is a single cone. This pattern is then reflected from the surface into a mirror that reflects the pattern into the camera.
Figure 7 shows a cross section of a model of the interior surface scanner according to the invention. Note that the camera has been moved out of the probe and a lens system is used to guide the image to the camera.
Figure 8 shows a cross section of a model of the interior surface scanner according to the invention. Note that the camera has been moved out of the probe and optical fibres are used to guide the image to the camera.
Figure 9 illustrates different positions sensors, which can be applied within the invention.
Figure 10 shows an embodiment of a hollow calibration object used for calibration of the camera and light sources. Note the symmetric 3D object feature curves on the object, which are utilised in the calibration.
Figure 11 shows a schematic sketch of a scanner according to the invention adapted for scanning of the ear and ear canal.
Figure 12 shows a schematic sketch of another embodiment of the scanner for the ear and ear canal.
Figure 13 shows a scan of an ear and an ear canal seen from two different views.
Figure 14 illustrates an embodiment of the scanner being able to scan the surface lying behind the end of the probe.

### 5. Detailed description of the invention

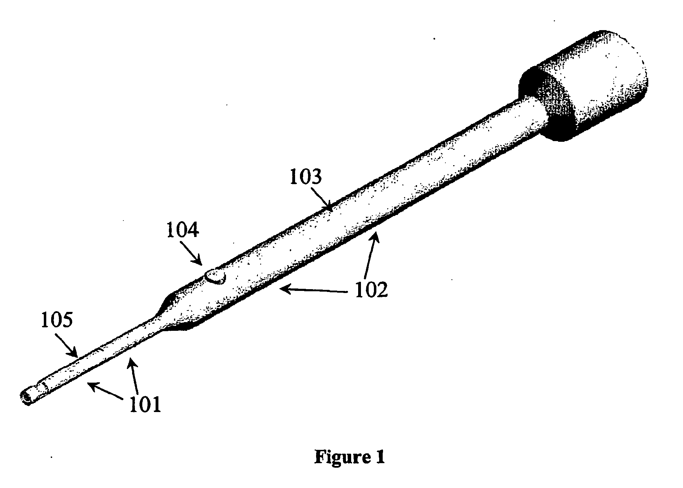

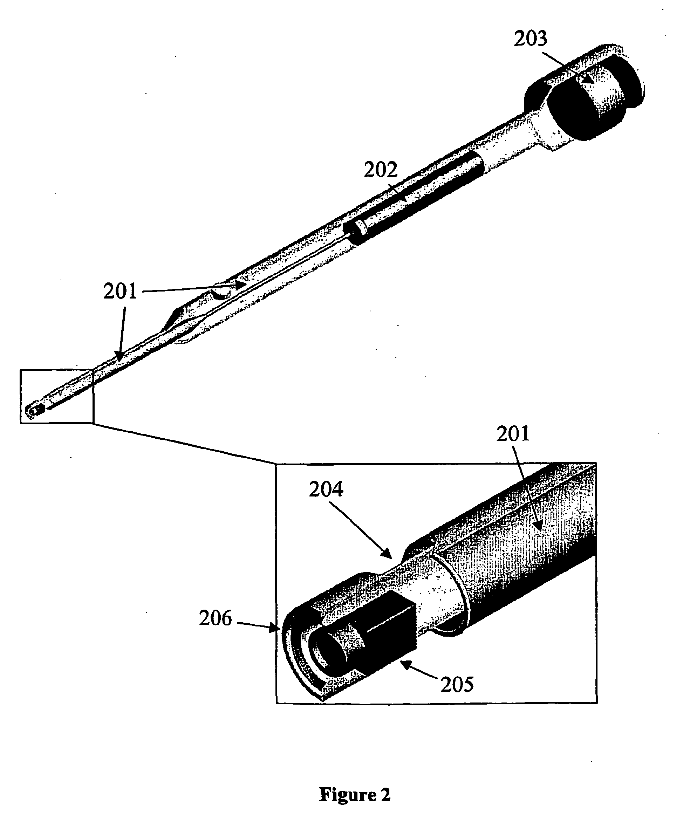

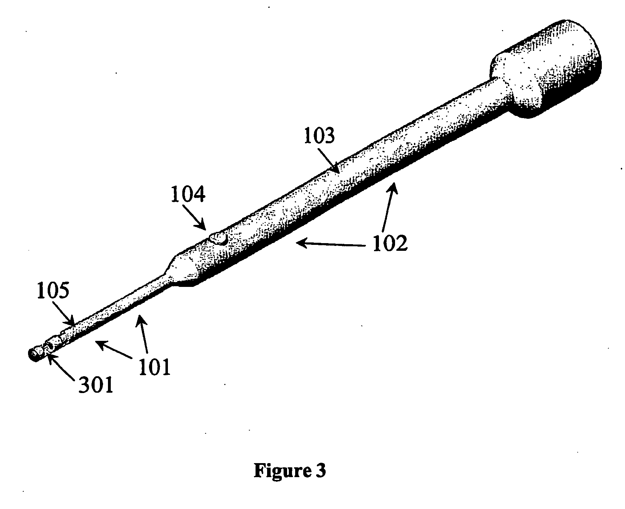

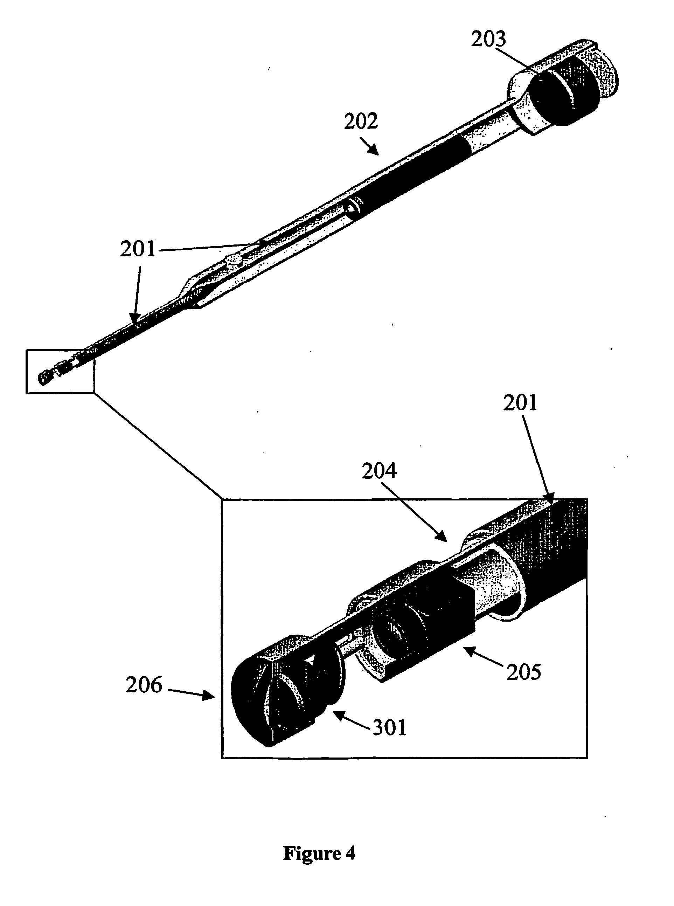

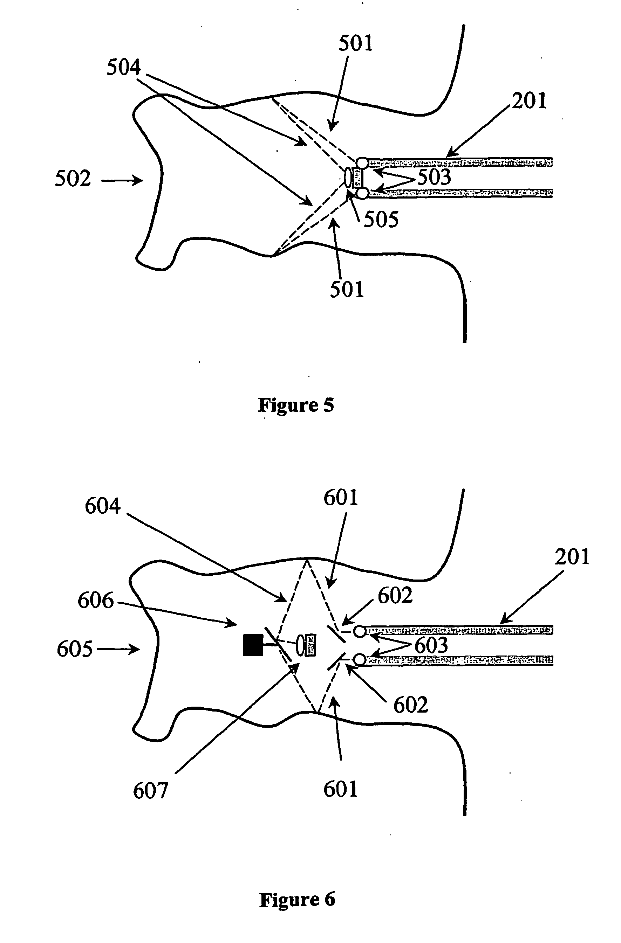

Figure 1 to Figure 4 illustrate two preferred embodiments of the invention. The first part 101 of the scanner is the probe, which is inserted into the cavity. The second part 102 is a handle. The scanner in Figure 1 and Figure 2 comprises a cover 103, a scan button 104, a disposable cover 105, light guides 201, a light source 202, a position sensor 203, optics and mirrors and/or prisms 204, a camera 205 and a protector/collision detector 206. A rotating mirror and/or prism with a micro motor 301 is also added to the component list in the embodiment shown in Figure 3 and Figure 4. As illustrated in Figure 5, the scanner works by projecting a structured light pattern 501 onto the interior surface of the object 502. The camera 503 acquires images of the reflection 504 of the light pattern from the surface. By locating the light pattern in the images, the corresponding 3D surface positions can be reconstructed applying well-known projective geometry. The scanner only scans limited parts of the surface at each position and usually it has to be moved around handheld or automatically to scan the full interior surface.

### 5.1 Light pattern generation

The light is generated by one or more light sources such as lasers, variable output-powered laser, light emitting diodes (LED), halogen spots or other spotlights and travels through the light guides such as optical fibres. In some applications it might be relevant to use monochromatic, coherent or polarised light. At the end of the light guides optics and mirrors and/or prisms may create the desired pattern. Examples of optics are filters, lenses or prisms. An alternative to the use of light guides is to place the light source near the tip of the scanner. Note that the projection of light, even lasers, onto the surface does not damage the surface.

The light sources for some applications preferably are as small as possible to minimise the dimensions of the scanner. It is thus contemplated that the light source may have a cross section perpendicular to the direction of emitted light of less than 5 mm, preferably less than 4 mm, for example less than 3 mm, such as less than 2 mm, for example less than 1 mm, such as less than 0.5 mm, for example less than 0.25 mm.

The scanner may work with only one light source, but for many purposes it is advantageous to have several such as at least 2 light sources, such as at least 3 light sources, for example at least 4 light sources, such as at least 5 light sources, such as at least 6 light sources, for example at least 7 light sources, such as at least 8 light sources, for example at least 10 light sources, such as at least 12 light sources, for example at least 16 light sources, such as at least 20 light sources.

Depending on the desired pattern one, two, three or more optics and one, two, three, four or more mirror and/or prisms are required. The structured light pattern may be a number of rays forming a grid of spots on the surface consisting of one, two, three, four or more rows of points, one, two, three or more cones of light forming contours on the surface, one, two, three of more planes of light forming contours on the surface, one, two, three of more thick planes of light forming thick contours on the surface, a number of rectangular shaped rays forming a distorted checker board pattern on the surface or more complex shapes.

Thus, when projecting a pattern of rays, pattern may comprise at least 10 rays, such as at least 25 rays, for example at least 100 rays, such as at least 1000 rays, for example at least 10,000 rays, such as at least 100,000 rays, for example at least 1,000,000 rays.

Figure 5 illustrates how a single light cone 501 is projected onto the object surface 502 using optics 503. Figure 6 shows how the emission angle of the light cone can be increased significantly by reflecting the emitted light 601 into a cone mirror and/or prism 602 after the optics 603. Any type of mirrors such as coplanar mirrors and cone mirrors can be used to reflect the light Applying mirrors and/or prisms make it possible to change the emission direction invariant of the orientation of the light guides. The light pattern can also be moved over the surface without moving the actual scanner by rotating and/or tilting the mirrors and/or prisms. The rotation and/or tilting of the mirrors and/or prisms may be carried out by a motor.

Preferably the location of the point of emission of light and the point of recording reflected light as well as the angle of emission and recording with respect to the axis of the probe are chosen to give an angle between incident light on the object and light reflected from the object of approximately 20-30°. An example of this embodiment is illustrated in figure 6.

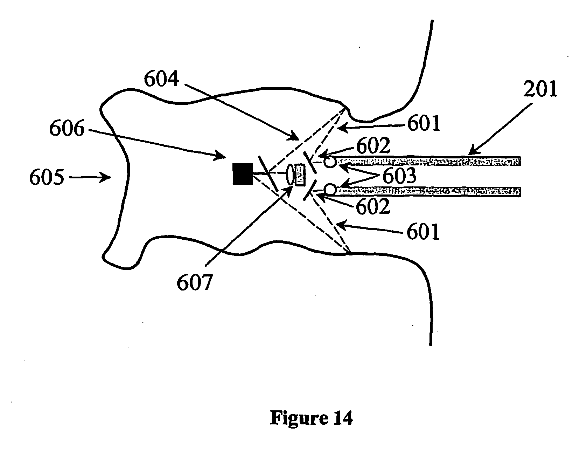

Occlusion effects represent a problem for some types of scanning of interior surfaces. Some of these can be overcome by selecting a direction of emission and recording of light with respect to the axis of the scanner, which ensures that light is projected on and recorded from all parts of the interior surfaces. One embodiment of the scanner is designed, wherein the location of the point of emission of light and the point of recording reflected light as well as the angle of emission and recording with respect to the axis of the probe are chosen to give a scan of the surface lying ahead of the end of the probe. An example of such a scanner is shown in Figure 5. Alternatively the location of the point of emission of light and the point of recording reflected light as well as the angle of emission and recording with respect to the axis of the probe may be chosen to give a scan of the surface lying approximately around the end of the probe. An example of this is shown in figure 6. Alternatively, the location of the point of emission of light and the point of recording reflected light as well as the angle of emission and recording with respect to the axis of the probe may be chosen to give a scan of the surface lying behind the end of the probe. Figure 14 illustrates an example of such a scanner. These alternative embodiments may be obtained with one scanner by tilting mirrors and/or prisms.

### 5.2 Light source intensity adjustment

The light source intensities are preferably varied depending on the surface and colour of the object to be scanned. Preferably the intensity should be determined automatically using automatic light source intensity calibration.

The intensity calibration may be performed by inserting the scanner into the object and calculate a number of histograms from the acquired images. First a histogram is calculated with the light source turned off. A second histogram is the calculated when the light source is turned on with an arbitrary intensity. The first histogram is then subtracted from the second to remove the background intensity. The intensity is then adjusted until the requested quantile corresponds to a predefined intensity. The background could also be removed by subtracting the image corresponding to the light source turned off from the image with light. The histogram used to determine the intensity could then be calculated from this difference image.

### 5.3 Image acquisition

The images are acquired by the one or more cameras. Preferably the cameras comprise a lens and a sensor array such as a CCD or CMOS chip. Usually the camera also comprises a filter placed in front of the sensor array. The effect of the filter is that only light with approximately the desired wavelength passes the filter. This makes it feasible to separate different light sources in the scanner and remove most of the background light. Alternatively, the camera may be colour sensitive.

The scanner may comprise just one camera or comprise several such as at least 2 cameras, such as at least 3 cameras, for example at least 4 cameras, such as at least 6 cameras, for example at least 7 cameras, such as at least 8 cameras, for example at least 10 cameras, such as at least 12 cameras, for example at least 16 cameras, such as at least 20 cameras.

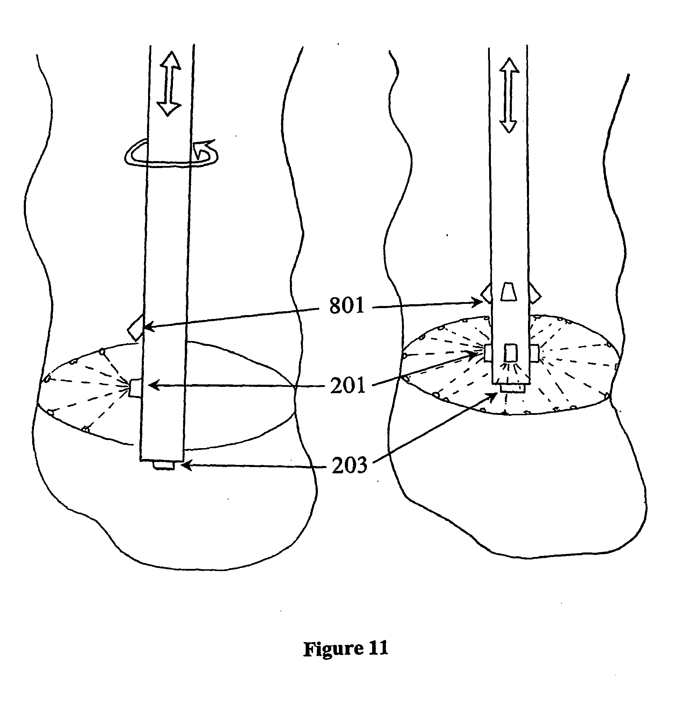

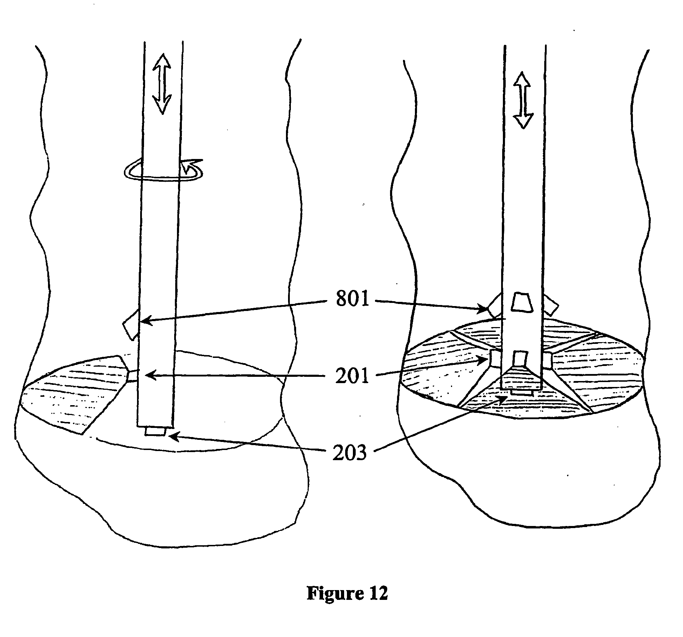

Preferably the cameras are arranged such that reflected light is recorded from different directions covering the 360° around the probe (Figure 11 and 12, right side).

Preferably the camera part of the scanner is as small as possible. The size of cameras is reduced almost every year, and it is estimated that the lower limit for camera size and pixel size have not been reached at all yet. Irrespective of the future development within this area, any camera smaller than the cameras presently available will be suitable for use in the present invention. Therefore the light detecting component of the camera may have a cross section in a direction perpendicular to the direction of incident light of less than 10 mm, such as less than 9 mm, for example less than 8 mm, such as less than 7 mm, for example less than 6 mm, such as less than 5 mm, for example less than 4 mm, such as less than 3 mm, for example less than 1 mm, such as less than 0.5 mm, for example less than 0.25 mm, such as less than 0.1 mm, for example less than 0.01 mm.

The number of pixels of the camera is a question of the size of the camera (depending on the size of the pixels), the computing power used for processing the results of the scans and the cost of the camera. No upper limit for the number of pixels can be set, since precision is increased whenever the number of pixels is increased. Accordingly, the camera may comprise an array of at least 125*125 pixels, more preferably at least 250*250 pixels, more preferably more than 500*500 pixels, more preferably more than 1000*1000 pixels, such as more than 2000*2000 pixels, for example more than 4000*4000 pixels, such as more than 8000*8000 pixels, for example more than 10,000*10,000 pixels, such as more than 25,000*25,000 pixels, for example more than 50,000*50,000 pixels, such as more than 100,000*100,000 pixels, for example more than 250,000*250,000 pixels, such as more than 500,000*500,000 pixels, for example more than 1,000,000*1,000,000 pixels. Similarly, the pixel size may be the smallest available on the market, for example wherein a cross section of a pixel is less than 100µm, such as less than 50 µm, for example less than 25 µm, such as less than 20µm, for example less than 15µm, such as less than 10µm, for example less than 7.5 µm, such as less than 5µm, for example less than 2.5 µm, such as less than 2 µm, for example less than 1.5 µm, such as less than 1 µm, for example less than 0.5 µ, such as less than 0.25 µm, for example less than 0.1 µm, such as less than 0.01 µm.

The light pattern may be reflected from the surface directly into the camera or into one or more light reflecting means such as mirrors or prisms before ending up in the camera. In the embodiment of the scanner in Figure 1 no mirrors are applied, since the scanner only needs to "look" forward with respect to the camera, i.e. the direction of the view is always parallel with the optical axis of the camera. Figure 5 illustrates the simple emission of the light pattern 501 and its reflections 504 from the object surface 502 into the camera 505 without the use of mirrors. Figure 5 is a simplified illustration of the principle used in the scanner in Figure 1.

Applying one or more mirrors and/or prisms for reflecting the light into the camera gives full freedom to select the direction of the view invariant of the orientation of the camera. Figure 6 illustrates how the emitted light pattern 601 is reflected using a cone mirror and/or prism 602 before it hits the object surface 605. The reflected light 604 is likewise reflected into a mirror and/or prism 606 before entering the camera 607. Figure 6 is a simplified illustration of the principle used in the scanner in Figure 3. Static mirrors such as coplanar or cone mirrors can be applied directly in the invention. Static mirrors have the advantage of being simple and mechanically stable.

In the embodiment of the scanner shown in Figure 3 the mirror and/or prism in front of the camera is coplanar, circular and able to rotate. The advantage of a rotating mirror and/or prism compared to a static mirror and/or prism, such as a cone mirror and/or prism, is that the image resolution and the field of view of the camera are significantly increased. Indeed resolution and field of view are seriously limited due to the small dimensions of the scanner, which directly affect the accuracy and flexibility. Tilting the mirror and/or prism further increases the accuracy and flexibility. In practice, the same mirror and/or prism can be used to generate the light pattern and reflecting the light into the camera. However, applying different mirrors and/or prisms for light and cameras, as presented in Figure 3, increase the flexibility of the scanner especially with respect to direction of view, depth of field and point reconstruction quality.

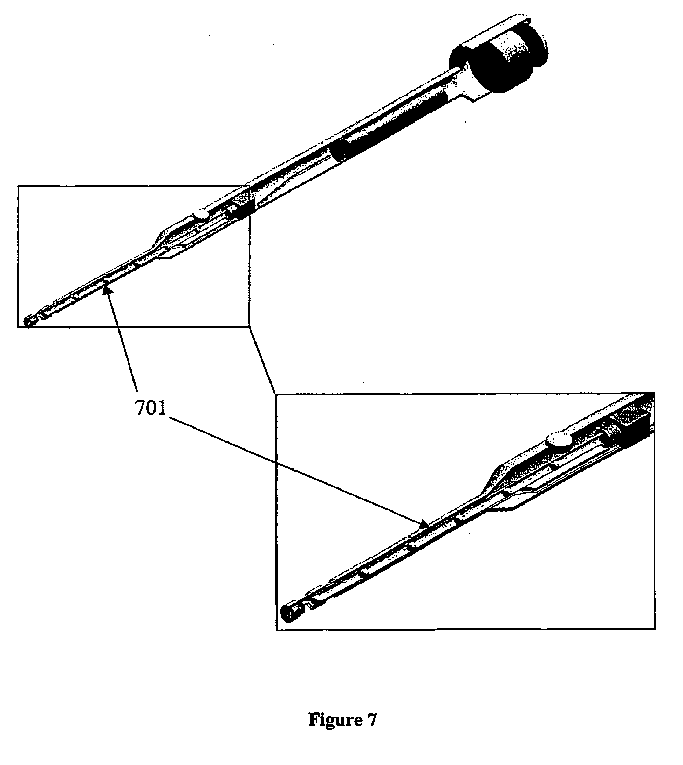

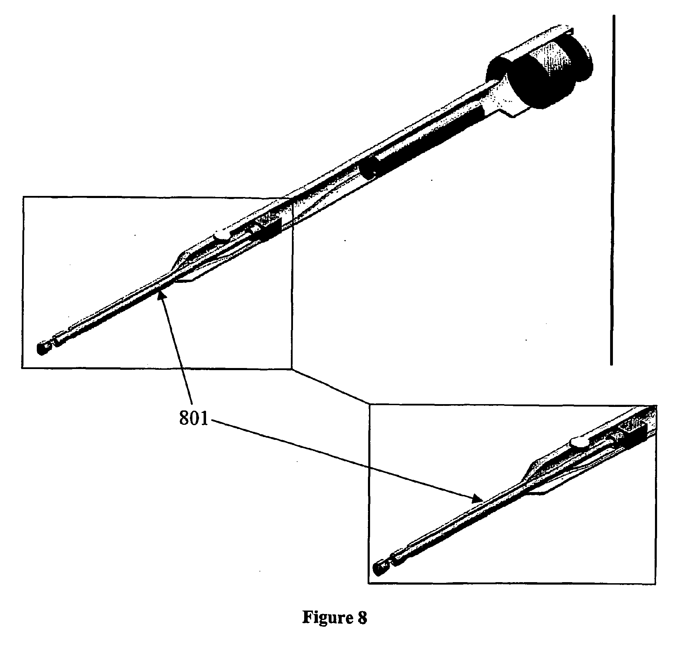

In the case of very small dimensions of the cavity and/or high requirements for accuracy it is infeasible to place the camera on the head of the scanner. The problem is solved by moving the cameras out of the probe. The image/light is then directed into the cameras by the use of light guides such as a lens system or optical fibres. An embodiment of the invention where a lens system 701 and optical fibres 801 are used as light guides are illustrated in Figure 7 and Figure 8, respectively. The lens system might be similar to the lens systems used in periscopes and endoscopes. At the moment the lens system is superior to optical fibres with respect to smallest dimensions and image quality. The disadvantage of the lens system is that it requires the probe be rigid, whereas the optical fibres are fully flexible, i.e. the probe can be flexible.

### 5.4 Position sensing

The objective of the position sensor is to determine the relative position and orientation of the probe head with respect to the object to be scanned. Knowing this position is extremely advantageous in combining the individual scans when the scanner or object is moved. Errors in the position measures will directly affect the quality of the scan. In the case of non-fixed objects such as the ear canal of humans are scanned, it is extremely advantageous to measure the position with respect to the object, e.g. the ear canal, and not to a fixed coordinate system, since the object might move during the scanning.

Recall that the position sensor is only required to combine the individual scans. The position sensor can be rendered superfluous by a registration of the individual scans. The output of the registration is the relative position of the scans. Knowing the relative positions of the scans make it straightforward to combine the scans. For the registration to be successful the interior surface needs to contain a proper number of distinct features, which is not always the case.

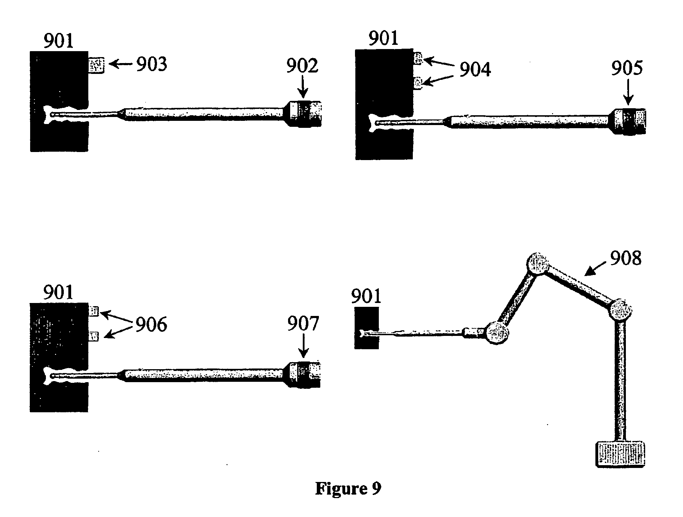

Preferably the position sensor should be a magnetic sensor as shown in Figure 9, where the receiver 902 usually is in the scanner and the transmitter 903 is secured to the object 901, e.g. the head of a human. Magnetic sensors have the advantage of not suffering for occlusion problems. Alternative sensors might be optical or sonic sensors. Figure 9 illustrates an optical sensor where markers 904 are placed on the object and a sensor 905 on the scanner. Likewise Figure 9 illustrates a sonic sensor, where an emitter 906 is placed on the object and a detector 907 is placed on the scanner. Both optical and sonic sensors suffer from occlusion problems, but their cost is often lower and the precision superior to those of magnetic sensors. In the case of a fixed object or an object, which can be fixed, a mechanical position sensor becomes attractive. As Illustrated in Figure 9 these sensors usually consist of a number of joints 908 connected by encoders. Many mechanical sensors are highly accurate, but they tend to be bulky or cumbersome to use.

In general, the position needs to be determined with respect to the head of the scanner. More precisely, the position of the focal point of the camera has to be determined when the camera is placed on the probe head. In the case where light guides are used in front of the camera, the position should correspond to the tip of the guides. With a rigid design of the scanner cover as in Figure 1 to Figure 4 the position sensor can be placed anywhere on the scanner, since the relative distance between the scan head and the position sensor is constant. With a flexible design of the probe the position sensor needs to be placed on the scan head, e.g. at the front as on the scanner in Figure 11 and Figure 12.

### 5.5 Cover

In the design of the scanner show in Figure 1 and Figure 3 only the probe 101 is supposed to move into the cavity. The main objective of the design has been to minimise the width of this part, since it determines the minimal size of the cavity, which can be scanned. In general the width of the probe can be varied freely down to approximately 0.1 mm, e.g. the width can be 30, 20, 15, 10, 8, 6, 5, 4, 3, 2, 1 or 0.1 mm. However the final design is a trade-off between size, accuracy and mechanical stability. In general the application determines the desirable design.

In the case of scanning the human ear canal the width of the part is requested to be below 4 mm. Figure 3 shows a scanner designed for scanning ear canals, where the width of the probe is 3.5 mm. The length of the probe can also be varied freely down to approximately 5 mm, e.g. the length can be 20, 35, 50, 100, 200, 300 or 500 mm. The length of the probe shown in Figure 1 and Figure 3 is 55 mm.

The rest of the scanner's cover is basically a handle. For optimal handling this part should preferably be 10-30 mm width and 100-150 mm long. The dimension can however be varied freely. As in Figure 1 and Figure 3 the width of the handle may be extended to make room for the components, e.g. the position sensor. The dimensions of this extension should however be minimised if the objective is to create the smallest and lightest scanner. The width and length of the extension shown in Figure 1 and Figure 3 is 40 mm and 30 mm, respectively. Note that larger light sources such as halogen spots may be moved to the extension.

In another embodiment of the scanner it is possible to rotate the probe 360 degrees around its axis. The advantage of this design compared to only rotating the mirrors and/or prisms as in Figure 3 is that the motor can be placed in the handle. Likewise another embodiment comprises a linear drive, which is able to translate the probe along its axis. The scanner can also be mounted on a robot, a magnetic position system or another device, which is able to position the scanner with any orientation and position within its workspace.

The choice of material for the cover depends on the actual application, especially whether the probe needs to be rigid or flexible. Preferably the cover should be produced in stainless steel or from a material selected from a group consisting of alloy, aluminium, a plastic polymer, kevlar®, ceramics or carbon.

In some application it might be necessary to protect the components such as cameras, mirrors and/or prisms and lenses against dust and other dirt. In practise this is done by inserting a window of transparent material such as glass or a plastic polymer in the holes in front of the relevant components.

### 5.6 Miscellaneous features

Other features in the preferred embodiment are a protector/collision detector, a scan button, and a disposable scanner cover. The protector consists of soft material such as rubber, silicone or a plastic polymer and ensures that the tip of the probe and the surface are not damaged in the case of a collision. In the case of scanning an ear canal it is crucial that the scanner does not damage the eardrum. In the case of very fragile surfaces, a collision detector adapted to measure the distance from the tip of the scanner to the bottom of the interior surface is added to the protector. When surfaces are scanned for which the scanner is subject to hygiene requirements, a disposable cover is desirable to minimize the need for cleaning. The disposable cover will usually only cover the probe or parts of it, but can be fit to the specific requirements. The scan button is used to start and stop the scan operation.

### 5.7 Processing

The acquired images are analysed real-time in a digital signal processor /microprocessor, which is placed in the scanner handle or in a separate processing box. The first step in the analysis of an image is to detect the light pattern in the image using a standard tracking algorithm. When the light pattern is known with subpixel precision, the corresponding 3D coordinates can be reconstructed using well-known projective geometry. A precise reconstruction of the 3D coordinates requires a very high quality of the camera and light calibration. The next step is to combine the 3D coordinates from different images acquired at the same or at different positions. The merging is simply performed by combining the individual points sets positioned with respect to their relative position. Finally the points are triangulated using a standard triangulation algorithm to form the final surface of the 3D model. The 3D model may then be transferred over a network to the destination for further use.

### 5.8 Uses of the scanner

The scanner according to the invention is especially adapted for scanning interior surfaces, such as body cavities and other interior surfaces with narrow openings, into which light from an external scanner cannot enter due to occlusion effects.

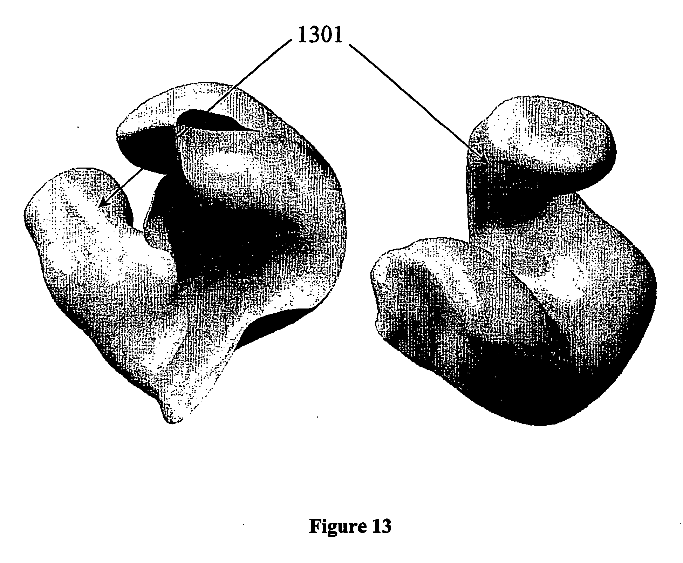

It is thus envisaged that the scanner is advantageous for scanning body cavities such as the internal surfaces of the ear, nose, mouth, teeth, stomach, lungs, alveoli, throat, rectum, vagina, veins, blood vessels, urinary tract. Scanning of teeth can be used in connection with correction of teeth and manufacture of dental implants. Scanning the blood vessels may be useful in connection with surgery. Scanning the vagina can be used in connection with pregnancy and delivery and also for measuring and modelling an individually adapted diaphragm. Figure 13 shows a scan of the interior surface of an ear and an ear canal 1301.

The scanner can also be used for industrial purposes such as for scanning internal surfaces of engines, fuel canals, bore, internal thread, pipes, tubes and containers. In this way the exact dimensions (volume and/or cross section and/or location of features) of the devices can be measured. When using a scanner with a position sensor this can be done more precisely than any of the known scanners. Furthermore, the present scanners are not sensitive to small deviations in the orientation of the axis of the scanner with respect to the axis of the object being scanned.

Another use is for archaeological purposes such as for scanning internal surfaces of jars, skulls and other archaeological items.

Furthermore, the scanners are very useful in industrial design especially in connection with computer assisted 3D modelling.

### 5.9 Scanning the ear

A possibility according to the invention is to scan the ear canal directly. This can be done by building the components of the scanner into an apparatus, which can be inserted into the ear of the patient. Embodiments of this scanner are shown in Figure 11. Preferably, the light source, e.g. the laser, and the camera are located outside the ear. The laser light can be carried into the scanner by light guides 201, and similarly, the reflected signals can be carried to a camera by another light guide 801. The scanner also consists of a position sensor 203, which measures the relative position of the scanner with respect to the object. During the scan, the scanner preferably rests on the edge of the ear canal, most preferably in those places where bones are closest to the skin surface. This is in order to obtain the highest stability and is very important, since the scanner itself works with an accuracy less than 0.05 mm. The length of the ear canal can be scanned by moving the scanner in or out and record a series of overlapping images of the ear canal. The scanner may comprise only one laser source and one camera as the one shown in the left of Figure 11. In that case the scanner has to rotate while the camera records images. Preferably, the scanner comprises multiple laser sources such as four as shown in the scanner in the right part of Figure 11. The presence of multiple laser sources and cameras removes the need for rotation of the scanner in the ear canal. In the laser scanner disclosed in Figure 11, the laser source or sources project a ray of laser light on the surface of the ear canal.

Another type of ear canal laser scanner is shown in Figure 12. Here the laser light is projected as laser sheets producing a laser contour on the surface of the ear canal. Thereby, more rapid scanning can be performed compared to the above laser scanner. In the scanner shown in the right part of Figure 12, four laser sheets and four cameras are present. Thereby the laser sheets cover the whole circumference and rotation of the scanner is not required.

The same types of variation of the ear canal scanner can be used as in other cases of three-dimensional scanners according to this invention. Thus, preferably the scanner comprises at least two cameras, more preferably 4 cameras such as for example 6 cameras. Likewise, there may be several laser sources such as for example 2 lasers creating laser sheets with an offset of 180°, preferably 3 laser sheets with an offset of 120°, or 4 laser sheets with an offset of 90°.

Prior to scanning, the patient's ear must be rinsed to remove cerumen. In some cases it may also be advantageous to treat the surface of the ear canal if the appearance is too glossy.

One scan can be performed in less than 1 minute, and it is thus possible to record a number of scans of one ear, and ask the patient to deliberately vary the size of the ear canal by swallowing, yawning, chewing, and drinking. In this way a series of scans of the ear canal can be recorded and the magnitude of the variation of the patient's ear canal can be detected. In the end it is possible to superimpose these scans on one another to create a model, which will fit the patient's ear under all conditions. Such a model is naturally made as a compromise between the different sizes of the ear canal.

The improved fit of the hearing aid shells according to the present invention compared to prior art hearing aid shells means that the frequent problem of acoustic feedback in hearing aids is minimised. The direct scanning of the ear significantly reduces the production cost of hearing aids, since the impressions used today are rendered superfluous. The removal of the impression removes the handling and mailing cost and cuts down the production time and improves flexibility.

### 5.10 Ear model

Currently hearing aids are created in a silicon mould, made with an ear impression.

It is possible to scan and create very detailed and accurate copies of ear impressions with the developed system as described in co-pending PCT/DK01/ ("Object and method for calibration of a three-dimensional light scanner", filed by 3-Shape on 24. August 2001).

Apart from hearing aids, other devices could also be inserted into a shell made to fit the ear canal of an individual. Such devices that could advantageously be incorporated into a shell manufactured according to the disclosed method include mobile phones, communication devices, loud speakers, tinnitus masking devices, or devices recording vibrations in the skull and transforming these into an audio signal.

Devices that may be incorporated into a shell in the ear also comprise devices related to Man Machine Interface (MMI) products, such as custom made ear microphone/receivers that enable reliable and clear communication even in the noisiest environments, or products related to wireless internet applications.

Speech not only creates sound waves, it also generates vibrations within the skull of the speaker. These vibrations can be picked up in the ear (they may be picked up other places too, but by far the most convenient method is to do it in the ear). In one piece, a device thus may comprise a microphone to pick up the speech of the person wearing it, and a loudspeaker to transmit the speech of the communication partner. It is important that such devices are made to fit the ear.

The devices based detection of vibration instead of sound can be used in the noisiest environments, since they only pick up the speech of the wearer and they allow for quiet communication, since the speaker can speak with a low voice when needed. The devices allow for completely hand-free communication.

Such a device is naturally also devoid of any kind of acoustic feedback if manufactured using the present invention.

### 5.11 Calibration

The precision of the light sources and cameras is very high today and so is that of the software developed to detect the intersection of the light sheet with the object and convert the two-dimensional data to three-dimensional co-ordinates. Therefore differences in precision and hence improvement of the precision primarily resides in the calibration of the systems. Recall that precision is of utmost importance in many applications.

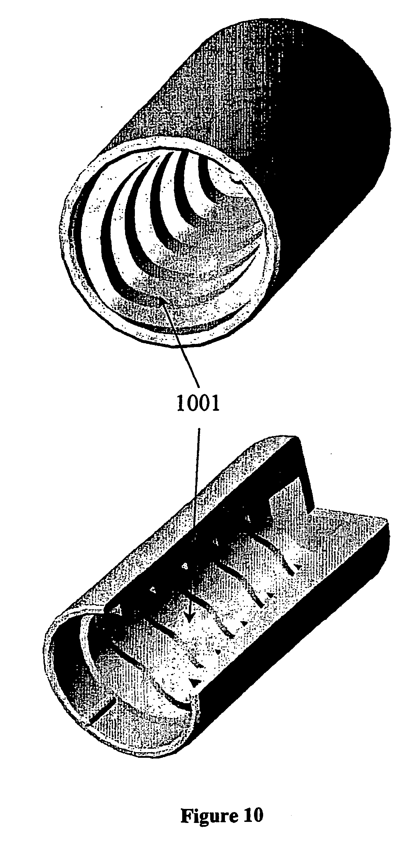

To obtain the highest precision both the light pattern and the camera need to be calibrated. Preferably the calibration should be performed using a calibration object with symmetrical 3D object feature curves and the corresponding methods as described below and in co-pending PCT/DK01/ ("Object and method for calibration of a three-dimensional light scanner", filed by 3-Shape on 24. August 2001). The main advantage of this type of calibration objects is that the light pattern can be calibrated independently of the calibration of the camera. An embodiment of the hollow calibration object used for calibration of the scanner is shown in Figure 10. Note the symmetric 3D object feature curves 1001 on the calibration object, which are utilised in the calibration.

Preferably, a light pattern is projected onto the calibration object to produce 2D image feature curves in the acquired images.

When preferred, the image feature curves may be determined using the Hough transformation, filter search, max intensity, threshold, centre of gravity, derivatives or other procedures.

The image feature coordinates are found as the intersection between image feature curves. These intersections could be seen in the images as corners or sharp edges of the image feature curves. The image feature co-ordinates may be found as the intersection between the image feature curves such as the intersection between two n^{th} order curves, as the intersection between two first order curves, as the intersection between two second order curves, as the intersection between two third order curves, as the intersection between a fist order curve and a second order curve, as the intersection between a first order curve and a third order curve, or as the intersection between a second order curve and a third order curve or as the intersection between any other possible combination of curves.

Preferably, the calibration method further comprises plotting of a mathematical combination of image feature points or features derived from these points against the angle of rotation or the translation of the calibration object. By plotting this function and optionally estimating a mathematical function describing the relationship between the function of an image co-ordinate and the angle of rotation or the translation, estimation of the light parameters and angle or rotation and/or translation becomes especially precise. The method may further comprise determination of the mean plane of symmetry in the plot.

The mean plane of symmetry can be determined by calculating the mean angle of rotation / mean translation for pairs of image feature points having the same value in the mathematical combination. Doing this produces multiple estimates for the encoder offset and light pattern displacement allowing also for the estimate of the laser sheet angle.

Light pattern calibration may also comprise selecting symmetric points, plotting of the rotation angle and/or the translation for the first point against the difference in the rotation angle and/or the translation between the two symmetric point, deriving mathematical formula for the plotted lines and estimating the light pattern parameters.

Alternatively, mathematical formulas can be derived for the curves, which appear in some of the plots of the mathematical combination as a function of the angle of rotation or the translation. Given these curves and the corresponding formulas, the encoder offset, the light pattern displacement and the light pattern angle can be estimated.

Preferably, light pattern co-ordinates of the 3D object feature curves are estimated corresponding to a discrete number of values of angle of rotation and/or translations. These values should preferably cover the whole circumference and/or length of the calibration object.

2D co-ordinates of the 3D object feature curves corresponding to a discrete number of values of angle or rotation and/or translation may be calculated from mathematical functions determining the 3D object feature curves. In order to determine the calibration parameters such as camera position, camera orientation, and camera optic parameters, pairs of 2D light pattern co-ordinates are compared to calculated 2D co-ordinates for a discrete number of values of angle or rotation and/or translation. This comparison preferably comprises using the Tsai or the Heikkilä algorithm. The advantage of the Tsai and the Heikkilä algorithm in this context is that it provides rapid and precise estimation of the calibration parameters such as radial lens distortion.

Alternative methods for calibration comprise direct linear transformation and direct non-linear matrix transformation optionally in combination with an optimisation procedure such as least squares means to minimise the error. In these cases initial calibration parameters may be estimated to facilitate the convergence of the parameters during optimisation.

To improve calibration precision outliers may be excluded from the calibration. Outliers can e.g. be identified in the plot of the mathematical combination of image feature coordinates against the angle of rotation / the translation or by back projection of co-ordinates after an initial calibration.

2% of the feature points deviating most from the back-projected 2D image feature curves may be excluded from the calibration or at least 3%, such as at least 5%, for example at least 10%, for example at least 12%, such as at least 15% for example at least 20, preferably at least 25%, for example at least 30 %, more preferably at least 33 % may be excluded to improve calibration precision.

In order to cover the whole circumference of the calibration object the discrete number of values for angle of rotation / translation may be at least 100, preferably at least 240, for example at least 500, such as at least 750, for example at least 1000, such as at least 1200, for example at least 1500, such as at least 1800, for example at least 2000, such as at least 2400, for example at least 3000, for example at least 3600, such as at least 4200. The higher the discrete number of values of angle of rotation / translation, the higher the calibration precision.

The highest calibration precision is obtained when using a rigid setup, which comprises mounting the calibration object onto mounting means.

### 6. References

[1] TSai, R. Y., "A versatile Camera Calibration Technique for High-Accuracy 3D Machine Vision Metrology Using Off-the-Shelf TV Cameras and Lenses", IEEE Journal of Robotics and Automation, pages 323-344, Vol. RA-3, No. 4, August 1987.
[2] Heikkilä, J, "Geometric Camera Calibration Using Circular Control Points", IEEE Transactions on Pattern Analysis and Machine Intelligence, Vol. 22, No. 10, pp. 1066-1077, Oct 2000

## Claims

1. A scanner for three-dimensional scanning of interior surfaces comprising
at least one light source adapted to create and project structured light producing a pattern on the interior surface of an object,
at least one camera, adapted to record 2-D images of the pattern,
data processing means adapted to convert 2-D image information into 3-D real world co-ordinates,
at least one position sensor adapted to provide the relative position and orientation of the scanner during successive scans,
the point of emission of light as well as the point of accumulation of reflected light for the camera being located on a probe having an axis,
the at least one light source and the at least one camera being adapted to perform a scan 360° around the axis, and
the probe being adapted to be entered into a cavity.

2. The scanner according to claim 1, wherein the probe is rigid.

3. The scanner according to claim 1, wherein the probe is flexible.

4. The scanner according to claim 1, wherein the probe has a diameter or cross section of less than 30 mm.

5. The scanner according to claim 1, wherein the probe has a diameter or cross section of less than 20 mm, preferably less than 15 mm, more preferably less than 10 mm, such as less than 10 mm, for example less than 8 mm, such as less than 6 mm, for example less than 5 mm, such as less than 4 mm, for example less than 3 mm, such as less than 2 mm, such as less than 1 mm, for example less than 0.1 mm.

6. The scanner according to claim 1, wherein the probe has a length of up to 500 mm, such as up to 300 mm, for example up to 200 mm, such as up to 100 mm, preferably up to 50 mm, more preferably up to 35 mm, such as up to 20 mm, for example up to 10 mm, such as up to 5 mm.

7. The scanner according to any of the preceding claims, wherein the probe is made from a material selected from the group consisting of an alloy, aluminium, kevlar, a polymer, ceramics, and carbon.

8. The scanner according to any of the preceding claims, wherein the probe is made from a material selected from the group consisting of polymers, such as plastic polymers, rubber, and silicone.

9. The scanner according to any of the preceding claims, wherein the pattern of structured light comprises at least one ray of light forming at least one point on the surface.

10. The scanner according to claim 9, wherein the pattern comprises at least 10 rays, such as at least 25 rays, for example at least 100 rays, such as at least 1000 rays, for example at least 10,000 rays, such as at least 100,000 rays, for example at least 1,000,000 rays.

11. The scanner according to any of the preceding claims, wherein the at least one light source is adapted to create and project rectangular shaped rays forming a distorted checkerboard pattern on the interior surface.

12. The scanner according to any of the preceding claims, wherein the pattern of structured light comprises at least one planes of light forming at least one contour on the surface.

13. The scanner according to any of the preceding claims, wherein the pattern of structured light comprises at least one cone of light.

14. The scanner according to claim 1, wherein the at least one light source comprises a laser, variable output-powered laser, light emitting diode (LED), a halogen spot or another spotlight, preferably being adapted to produce monochromatic and/or coherent and/or polarised light.

15. The scanner according to claim 1, wherein the light source has a cross section perpendicular to the direction of emitted light of less than 5 mm, preferably less than 4 mm, for example less than 3 mm, such as less than 2 mm, for example less than 1 mm, such as less than 0.5 mm, for example less than 0.25 mm.

16. The scanner according to claim 1, further comprising optics such as filters, lenses or prisms to create and/or focus the pattern of light on the surface of the object.

17. The scanner according to claim 1, comprising at least 2 light sources, such as at least 3 light sources, for example at least 4 light sources.

18. The scanner according to claim 17 comprising at least 5 light sources, such as at least 6 light sources, for example at least 7 light sources, such as at least 8 light sources, for example at least 10 light sources, such as at least 12 light sources, for example at least 16 light sources, such as at least 20 light sources.

19. The scanner according to any of the preceding claims, whereby the light source intensity is varied depending on the surface and/or colour of the object to be scanned.

20. The scanner according to claim 19, whereby the light source intensity is determined automatically using automatic light source intensity calibration.

21. The scanner according to any of the preceding claims, wherein the at least one light source is arranged such that structured light is emitted in different directions covering 360° around the probe.

22. The scanner according to any of the preceding claims, wherein the at least one camera comprises a sensor array.

23. The scanner according to any of the preceding claims, wherein the at least one camera comprises a CCD.

24. The scanner according to any of the preceding claims, wherein the at least one camera comprises a CMOS.

25. The scanner according to any of the preceding claims, wherein the light detecting component of the camera has a cross section in a direction perpendicular to the direction of incident light of less than 10 mm, such as less than 9 mm, for example less than 8 mm, such as less than 7 mm, for example less than 6 mm, such as less than 5 mm, for example less than 4 mm, such as less than 3 mm, for example less than 1 mm, such as less than 0.5 mm, for example less than 0.25 mm, such as less than 0.1 mm, for example less than 0.01 mm.

26. The scanner according to any of the preceding claims, wherein the camera comprises an array of at least 125*125 pixels, more preferably at least 250*250 pixels, more preferably more than 500*500 pixels, more preferably more than 1000*1000 pixels, such as more than 2000*2000 pixels, for example more than 4000*4000 pixels, such as more than 8000*8000 pixels, for example more than 10,000*10,000 pixels, such as more than 25,000*25,000 pixels, for example more than 50,000*50,000 pixels, such as more than 100,000*100,000 pixels, for example more than 250,000*250,000 pixels, such as more than 500,000*500,000 pixels, for example more than 1,000,000*1,000,000 pixels.

27. The scanner according to claim 26, wherein the pixel size is the smallest available on the market, for example wherein a cross section of a pixel is less than 100µm, such as less than 50 µm, for example less than 25 µm, such as less than 20µm, for example less than 15µm, such as less than 10µm, for example less than 7.5 µm, such as less than 5µm, for example less than 2.5 µm, such as less than 2 µm, for example less than 1.5 µm, such as less than 1 µm, for example less than 0.5 µ, such as less than 0.25 µm, for example less than 0.1 µm, such as less than 0.01 µm.

28. The scanner according to any of the preceding claims, wherein the at least one camera is colour sensitive.

29. The scanner according to any of the preceding claims, further comprising a filter in the light path between the object surface and the camera being adapted to filter light from other light sources than the individual light sources of the scanner.

30. The scanner according to claim 1, comprising at least 2 cameras, such as at least 3 cameras, for example at least 4 cameras.

31. A method for scanning interior surfaces comprising the steps of
i) entering a probe shaped scanner having an axis into a cavity,
ii) creating and projecting structured light from a first point on the probe producing a pattern on an interior surface of an object, and at a second point of the probe, recording 2D images of the pattern reflected from the interior surface, thereby performing a scan 360° around the axis of the probe,
iii) determining 2D co-ordinates of the images of the pattern,
iv) determining the relative position and orientation of the scanner during successive scans with at least one position sensor,
v) combining a series of images to obtain 3D real world co-ordinates of the interior surface.

32. The method according to claim 31, wherein the scanner is as defined in claim 1.

33. The method according to any of claims 31 to 32, further comprising calibration of the scanner
i) scanning a three dimensional calibration object having at least one plane of symmetry and whereby at least part of at least one 3D object feature curve of each symmetric part is a continuous curve,
ii) determining image feature co-ordinates being representations of at least one pair of 3D object feature curves for each of a discrete number of values of an angle of rotation and/or a translation, a pair consisting of one 3D object feature curve in each symmetric part of the calibration object,
iii) changing the calibration parameters to fit the calibration object.

34. The method according to claim 33, whereby a light pattern is projected onto the calibration object producing 2D image feature curves.

35. The method according to claim 34, whereby the 2D image feature curves are determined using the Hough transformation.

36. The method according to claim 33, whereby 2D image feature co-ordinates are found as the intersection between image feature curves such as, the intersection between two n^{th} order curves, as the intersection between two first order curves, as the intersection between two second order curves, as the intersection between two third order curves, as the intersection between a first order curve and a second order curve, as the intersection between a first order curve and a third order curve, or as the intersection between a second order curve and a third order curve.

37. A method for 3D modelling and production comprising
obtaining 3D real world coordinates of an interior surface of a cavity provided using the method according to any of claims 31 to 33, and
creating a piece adapted to fit into the cavity.

38. The method according to claim 37, wherein production comprises milling, 3-dimensional printing, stereo lithography, selective laser sintering, laminated object modelling, inkjet modelling, fused deposition modelling, nano-printing.

39. The method according to any of claims 37 to 38, wherein 3D modelling comprising computer assisted modelling of the scan data prior to production.

40. The method according to any of the preceding claims 37 to 39, comprising scanning the interior surface of an ear canal and producing a hearing aid shell adapted to house a device.

## Patentansprüche

1. Scanner zum dreidimensionalen Scannen innerer Oberflächen, umfassend
wenigstens eine Lichtquelle, die dazu ausgelegt ist, strukturiertes Licht zu erzeugen und zu projizieren, das ein Muster auf der inneren Oberfläche eines Objektes erzeugt,
wenigstens eine Kamera, die dazu ausgelegt ist, 2D-Bilder des Musters zu erzeugen,
Datenverarbeitungsmittel, die dazu ausgelegt sind, 2D-Bildinformation in 3D-Echtraumkoordinaten zu konvertieren,
wenigstens einen Lagesensor, der dazu ausgelegt ist, die relative Position und Orientierung des Scanners während aufeinanderfolgender Scans zur Verfügung zu stellen,
wobei der Ort der Lichtemission und der Ort der Akkumulation reflektierten Lichtes für die Kamera auf einer Sonde mit einer Achse angeordnet sind,
wobei die wenigstens eine Lichtquelle und die wenigstens eine Kamera dazu ausgelegt sind, einen 360°-Scan um die Achse auszuführen und
wobei die Sonde dazu ausgelegt ist, in einen Hohlraum eingeführt zu werden.

2. Scanner nach Anspruch 1, wobei die Sonde starr ist.

3. Scanner nach Anspruch 1, wobei die Sonde flexibel ist.

4. Scanner nach Anspruch 1, wobei die Sonde einen Durchmesser oder Querschnitt von weniger als 30 mm hat.

5. Scanner nach Anspruch 1, wobei die Sonde einen Durchmesser oder Querschnitt von weniger 20 mm, vorzugsweise weniger als 15 mm, weiter bevorzugt weniger als 10 mm, wie weniger als 10 mm, z.B. weniger als 8 mm, wie weniger als 6 mm, z.B. weniger als 5 mm, wie weniger als 4 mm, z.B. weniger als 3 mm, wie weniger als 2 mm, wie weniger als 1 mm, z.B. weniger als 0,1 mm hat.

6. Scanner nach Anspruch 1, wobei die Sonde eine Länge von bis zu 500 mm, wie bis zu 300 mm, z.B. bis zu 200 mm, wie bis zu 100 mm, vorzugsweise bis zu 50 mm, weiter bevorzugt bis zu 35 mm, wie bis zu 20 mm, z.B. bis zu 10 mm, wie bis zu 5 mm hat.

7. Scanner nach einem der vorangehenden Ansprüche, wobei die Sonde aus einem Material hergestellt ist, das aus der Gruppe ausgewählt ist, die aus einer Legierung, Aluminium, Kevlar, einem Polymer, Keramik und Kohlenstoff besteht.

8. Scanner nach einem der vorangehenden Ansprüche, wobei die Sonde aus einem Material hergestellt ist, das aus der Gruppe ausgewählt ist, die aus Polymeren, wie Kunststoffpolymeren, Gummi und Silikon besteht.

9. Scanner nach einem der vorangehenden Ansprüche, wobei das Muster des strukturierten Lichtes wenigstens einen Lichtstrahl umfaßt, der wenigstens einen Punkt auf der Oberfläche bildet.

10. Scanner nach Anspruch 9, wobei das Muster wenigstens 10 Strahlen, wie wenigstens 25 Strahlen, z.B. wenigstens 100 Strahlen, wie wenigstens 1.000 Strahlen, z.B. wenigstens 10.000 Strahlen, wie wenigstens 100.000 Strahlen, z.B. wenigstens 1.000.000 Strahlen umfaßt.

11. Scanner nach einem der vorangehenden Ansprüche, wobei die wenigstens eine Lichtquelle dazu ausgelegt ist, rechteckig geformte Strahlen zu erzeugen und zu projizieren, die ein verzerrtes Schachbrettmuster auf der inneren Oberfläche bilden.

12. Scanner nach einem der vorangehenden Ansprüche, wobei das Muster strukturierten Lichtes wenigstens eine Ebene von Licht umfaßt, die wenigstens eine Kontur auf der Oberfläche bildet.

13. Scanner nach einem der vorangehenden Ansprüche, wobei das Muster strukturierten Lichtes wenigstens einen Lichtkegel umfaßt.

14. Scanner nach Anspruch 1, wobei die wenigstens eine Lichtquelle einen Laser, einen Laser mit variabler Ausgabeenergieversorgung, eine lichtemittierende Diode (LED), einen Halogenpunktstrahler oder einen anderen Punktstrahler umfaßt, der vorzugsweise dazu ausgelegt ist, monochromatisches und/oder kohärentes und/oder polarisiertes Licht zu erzeugen.

15. Scanner nach Anspruch 1, wobei die Lichtquelle einen Querschnitt rechtwinklig zur Richtung des emittierten Lichtes von weniger als 5 mm, vorzugsweise weniger als 4 mm, z.B. weniger als 3 mm, wie weniger als 2 mm, z.B. weniger als 1 mm, wie weniger als 0,5 mm, z.B. weniger als 0,25 mm hat.

16. Scanner nach Anspruch 1, ferner umfassend optische Elemente wie Filter, Linsen oder Prismen, um das Lichtmuster auf der Oberfläche des Objektes zu erzeugen und/oder zu fokussieren.

17. Scanner nach Anspruch 1, umfassend wenigstens 2 Lichtquellen, wie wenigstens 3 Lichtquellen, z.B. wenigstens 4 Lichtquellen.

18. Scanner nach Anspruch 17, umfassend wenigstens 5 Lichtquellen, wie wenigstens 6 Lichtquellen, z.B. wenigstens 7 Lichtquellen, wie wenigstens 8 Lichtquellen, z.B. wenigstens 10 Lichtquellen, wie wenigstens 12 Lichtquellen, z.B. wenigstens 16 Lichtquellen, wie wenigstens 20 Lichtquellen.

19. Scanner nach einem der vorangehenden Ansprüche, wobei die Intensität der Lichtquelle in Abhängigkeit von der Oberfläche und/oder der Farbe des zu scannenden Objektes variiert wird.

20. Scanner nach Anspruch 19, wobei die Intensität der Lichtquelle automatisch unter Verwendung einer automatischen Lichtquellenintensitätskalibrierung bestimmt wird.

21. Scanner nach einem der vorangehenden Ansprüche, wobei die wenigstens eine Lichtquelle so angeordnet ist, daß strukturiertes Licht in verschiedene Richtungen, die 360° um die Sonde abdecken, emittiert wird.

22. Scanner nach einem der vorangehenden Ansprüche, wobei die wenigstens eine Kamera ein Sensorfeld umfaßt.

23. Scanner nach einem der vorangehenden Ansprüche, wobei die wenigstens eine Kamera ein CCD umfaßt.

24. Scanner nach einem der vorangehenden Ansprüche, wobei die wenigstens eine Kamera ein CMOS umfaßt.

25. Scanner nach einem der vorangehenden Ansprüche, wobei die lichtdetektierende Komponente der Kamera einen Querschnitt in einer Richtung rechtwinklig zur Richtung des einfallenden Lichtes von weniger als 10 mm, wie weniger als 9 mm, z.B. weniger als 8 mm, wie weniger als 7 mm, z.B. weniger als 6 mm, wie weniger als 5 mm, z.B. weniger als 4 mm, wie weniger als 3 mm, z.B. weniger als 1 mm, wie weniger als 0,5 mm, z.B. weniger als 0,25 mm, wie weniger als 0,1 mm, z.B. weniger als 0,01 mm hat.

26. Scanner nach einem der vorangehenden Ansprüche, wobei die Kamera ein Feld von wenigstens 125*125 Pixeln, bevorzugter wenigstens 250*250 Pixeln, bevorzugter mehr als 500*500 Pixeln, bevorzugter mehr als 1.000*1.000 Pixeln, wie mehr als 2.000*2.000 Pixeln, z.B. mehr als 4.000*4.000 Pixeln, wie mehr als 8.000*8.000 Pixeln, z.B. mehr als 10.000*10.000 Pixeln, wie mehr als 25.000*25.000 Pixeln, z.B. mehr als 50.000*50.000 Pixeln, wie mehr als 100.000*100.000 Pixeln, z.B. mehr als 250.000*250.000 Pixeln, wie mehr als 500.000*500.000 Pixeln, z.B. mehr als 1.000.000*1.000.000 Pixeln hat.

27. Scanner nach Anspruch 26, wobei die Pixelgröße die kleinste auf dem Markt erhältliche ist, z.B. wobei ein Querschnitt eines Pixels weniger als 100 µm, wie weniger als 50 µm, z.B. weniger als 25 µm, wie weniger als 20 µm, z.B. weniger als 15 µm, wie weniger als 10 µm, z.B. weniger als 7,5 µm, wie weniger als 5 µm, z.B. weniger als 2,5 µm, wie weniger als 2 µm, z.B. weniger als 1,5 µm, wie weniger als 1 µm, z.B. weniger als 0,5 µm, wie weniger als 0,25 µm, z.B. weniger als 0,1 µm, wie weniger als 0,01 µm beträgt.

28. Scanner nach einem der vorangehenden Ansprüche, wobei die wenigstens eine Kamera farbempfindlich ist.

29. Scanner nach einem der vorangehenden Ansprüche, ferner umfassend einen Filter in dem Lichtweg zwischen der Objektoberfläche und der Kamera, der dazu ausgelegt ist, Licht von anderen Lichtquellen als den individuellen Lichtquellen des Scanners auszufiltern.

30. Scanner nach Anspruch 1, umfassend wenigstens 2 Kameras, wie wenigstens 3 Kameras, z.B. wenigstens 4 Kameras.

31. Verfahren zum Scannen innerer Oberflächen, umfassend die Schritte des
i) Einführens eines sondenförmigen Scanners mit einer Achse in einen Hohlraum,
ii) Erzeugens und Projizierens strukturierten Lichtes von einem ersten Ort auf der Sonde, das ein Muster auf einer inneren Oberfläche eines Objektes erzeugt, und Aufzeichnens von 2D-Bildern des von der inneren Oberfläche reflektierten Musters an einer zweiten Stelle der Sonde, wodurch ein 360°-Scan um die Achse der Sonde ausgeführt wird,
iii) Bestimmens von 2D-Koordinaten der Bilder des Musters,
iv) Bestimmens der relativen Position und Orientierung des Scanners während aufeinanderfolgender Scans mittels wenigstens eines Positionssensors,
v) Kombinierens einer Reihe von Bildern, um 3D-Echtraumkoordinaten der inneren Oberfläche zu erhalten.

32. Verfahren nach Anspruch 31, wobei der Scanner wie in Anspruch 1 definiert ist.

33. Verfahren nach einem der Ansprüche 31 oder 32, ferner umfassend die Kalibrierung des Scanners durch
i) Scannen eines dreidimensionalen Kalibrierobjektes, das wenigstens eine Symmetrieebene hat und wobei wenigstens eine 3D-Objekteigenschaftskurve jedes Symmetrieteils eine symmetrische Kurve ist,
ii) Bestimmen von Bildeigenschaftskoordinaten, die Darstellungen von wenigstens einem Paar von 3D-Objekteigenschaftskurven für jeden einer diskreten Anzahl von Werten eines Drehwinkels und/oder einer Translation sind, wobei ein Paar aus einer 3D-Objektseigenschaftskurve in jedem Symmetrieteil des Kalibrierungsobjektes besteht,
iii) Ändern der Kalibrierparameter, um dem Kalibrierungsobjekt zu passen.

34. Verfahren nach Anspruch 33, wobei ein Lichtmuster auf das Kalibrierungsobjekt projiziert wird, das 2D-Bildeigenschaftskurven erzeugt.

35. Verfahren nach Anspruch 34, wobei die 2D-Bildeigenschaftskurven unter Verwendung der Hough-Transformation bestimmt werden.

36. Verfahren nach Anspruch 33, wobei die Koordinaten von 2D-Bildeigenschaften als Schnittpunkte zwischen Bildeigenschaftskurven wie dem Schnittpunkt zwischen zwei Kurven n-ter Ordnung, als Schnittpunkt zwischen zwei Kurven erster Ordnung, als Schnittpunkt zwei Kurven zweiter Ordnung, als Schnittpunkt zwischen zwei Kurven dritter Ordnung, als Schnittpunkt zwischen einer Kurve erster Ordnung und einer Kurve zweiter Ordnung, als Schnittpunkt zwischen einer Kurve erster Ordnung und einer Kurve dritter Ordnung oder als Schnittpunkt zwischen einer Kurve zweiter Kurve und einer Kurve dritter Ordnung gefunden werden.

37. Verfahren zum 3D-Modellieren und Herstellen umfassend
Erhalten von 3D-Echtraumkoordinaten einer inneren Oberfläche, die unter Verwendung des Verfahrens nach einem der Ansprüche 31 - 33 zur Verfügung gestellt werden, und
Herstellen eines Teils, das dazu ausgelegt ist, in den Hohlraum zu passen.

38. Verfahren nach Anspruch 37, wobei das Herstellen Fräsen, dreidimensionales Drucken, Stereolithografie, selektives Lasersintern, geschichtetes Objektmodellieren, Tintenstrahlmodellieren, Schmelz-Ablagerungs-Modellieren und Nanodrucken umfaßt.

39. Verfahren nach einem der Ansprüche 37 - 38, wobei das 3D-Modellieren computergestütztes Modellieren der Scandaten vor dem Herstellen umfaßt.

40. Verfahren nach einem der Ansprüche 37 - 39, umfassend das Scannen der inneren Oberfläche eines Ohrkanals und Herstellen einer Hörgeräteschale, die dazu ausgelegt ist, eine Vorrichtung aufzunehmen.

## Revendications

1. Scanner pour le balayage tridimensionnel de surfaces intérieures comprenant au moins une source lumineuse conçue pour créer et projeter une lumière structurée produisant un motif sur la surface intérieure d'un objet,
au moins une caméra, conçue pour enregistrer des images en 2-D du motif,
des moyens de traitement de données conçus pour convertir les informations d'images en 2-D en coordonnées universelles réelles en 3-D,
au moins un capteur de position conçu pour donner la position et l'orientation relatives du scanner au cours de balayages successifs,
le point d'émission de lumière ainsi que le point d'accumulation de la lumière réfléchie pour la caméra étant situés sur une sonde ayant un axe,
l'au moins une source de lumière et l'au moins une caméra étant conçues pour effectuer un balayage de 360° autour de l'axe, et
la sonde étant conçue pour être insérée dans une cavité.

2. Scanner selon la revendication 1, dans lequel la sonde est rigide.

3. Scanner selon la revendication 1, dans lequel la sonde est flexible.

4. Scanner selon la revendication 1, dans lequel la sonde a un diamètre ou une section transversale inférieur(e) à 30 mm.

5. Scanner selon la revendication 1, dans lequel la sonde a un diamètre ou une section transversale inférieur(e) à 20 mm, de préférence inférieur (e) à 15 mm, de manière davantage préférée inférieur(e) à 10 mm, comme inférieur(e) à 10 mm, par exemple, inférieur(e) à 8 mm, comme inférieur(e) à 6 mm, par exemple, inférieur(e) à 5 mm, comme inférieur(e) à 4 mm, par exemple, inférieur(e) à 3 mm, comme inférieur(e) à 2 mm, comme inférieur(e) à 1 mm, par exemple, inférieur(e) à 0,1 mm.

6. Scanner selon la revendication 1, dans lequel la sonde a une longueur pouvant aller jusqu'à 500 mm, comme jusqu'à 300 mm, par exemple, jusqu'à 200 mm, comme jusqu'à 100 mm, de préférence jusqu'à 50 mm, de manière davantage préférée jusqu'à 35 mm, comme jusqu'à 20 mm, par exemple, jusqu'à 10 mm, comme jusqu'à 5 mm.

7. Scanner selon l'une quelconque des revendications précédentes, dans lequel la sonde est réalisée à partir d'un matériau choisi dans le groupe comprenant un alliage, de l'aluminium, du kevlar, un polymère, de la céramique et du carbone.

8. Scanner selon l'une quelconque des revendications précédentes, dans lequel la sonde est réalisée à partir d'un matériau choisi dans le groupe comprenant les polymères, tels que les polymères plastiques, le caoutchouc et la silicone.

9. Scanner selon l'une quelconque des revendications précédentes, dans lequel le motif de la lumière structurée comprend au moins un rayon de lumière formant au moins un point sur la surface.

10. Scanner selon la revendication 9, dans lequel le motif comprend au moins 10 rayons, comme au moins 25 rayons, par exemple, au moins 100 rayons, comme au moins 1000 rayons, par exemple, au moins 10 000 rayons, comme au moins 100 000 rayons, par exemple, au moins 1 000 000 rayons.

11. Scanner selon l'une quelconque des revendications précédentes, dans lequel l'au moins une source de lumière est conçue pour créer et projeter des rayons de forme rectangulaire formant un motif en damier déformé sur la surface intérieure.

12. Scanner selon l'une quelconque des revendications précédentes, dans lequel le motif de la lumière structurée comprend au moins un plan de lumière formant au moins un contour sur la surface.

13. Scanner selon l'une des revendications précédentes, dans lequel le motif de la lumière structurée est composé d'au moins un cône de lumière.

14. Scanner selon la revendication 1, dans lequel l'au moins une source de lumière comprend un laser, un laser à puissance de sortie variable, une diode électroluminescente (LED), un spot halogène ou un autre spot, conçu de préférence pour produire une lumière monochrome et/ou cohérente et/ou polarisée.

15. Scanner selon la revendication 1, dans lequel la source de lumière a une section transversale perpendiculaire à la direction de la lumière émise inférieure à 5 mm, de préférence, inférieure à 4 mm, par exemple, inférieure à 3 mm, comme inférieure à 2 mm, par exemple, inférieure à 1 mm, comme inférieure à 0,5 mm, par exemple, inférieure à 0,25 mm.

16. Scanner selon la revendication 1, comprenant en outre des optiques telles que filtres, lentilles ou prismes pour créer et/ou focaliser le motif de lumière sur la surface de l'objet.

17. Scanner selon la revendication 1, comprenant au moins 2 sources de lumière, comme au moins 3 sources de lumière, par exemple, au moins 4 sources de lumière.

18. Scanner selon la revendication 17, comprenant au moins 5 sources de lumière, comme au moins 6 sources de lumière, par exemple, au moins 7 sources de lumière, comme au moins 8 sources de lumière, par exemple, au moins 10 sources de lumière, comme au moins 12 sources de lumière, par exemple, au moins 16 sources de lumière, comme au moins 20 sources de lumière.

19. Scanner selon l'une quelconque des revendications précédentes, dans lequel l'intensité de la source de lumière varie en fonction de la surface et/ou de la couleur de l'objet devant être scanné.

20. Scanner selon la revendication 19, dans lequel l'intensité de la source de lumière est déterminée automatiquement au moyen de l'étalonnage d'intensité de source de lumière automatique.

21. Scanner selon l'une quelconque des revendications précédentes, dans lequel l'au moins une source de lumière est agencée de telle sorte que la lumière structurée est émise dans des directions différentes en couvrant 360° autour de la sonde.

22. Scanner selon l'une quelconque des revendications précédentes, dans lequel l'au moins une caméra comprend un réseau de capteurs.

23. Scanner selon l'une quelconque des revendications précédentes, dans lequel l'au moins une caméra comprend un CCD.

24. Scanner selon l'une quelconque des revendications précédentes, dans lequel l'au moins une caméra comprend un CMOS.

25. Scanner selon l'une quelconque des revendications précédentes, dans lequel le composant de détection de lumière de la caméra a une section transversale dans une direction perpendiculaire à la direction de la lumière incidente inférieure à 10 mm, comme inférieure à 9 mm, par exemple, inférieure à 8 mm, comme inférieure à 7 mm, par exemple, inférieure à 6 mm, comme inférieure à 5 mm, par exemple, inférieure à 4 mm, comme inférieure à 3 mm, par exemple, inférieure à 1 mm, comme inférieure à 0,5 mm, par exemple, inférieure à 0,25 mm, comme inférieure à 0,1 mm, par exemple, inférieure à 0,01 mm.

26. Scanner selon l'une quelconque des revendications précédentes, dans lequel la caméra comprend un réseau d'au moins 125*125 pixels, de préférence, d'au moins 250*250 pixels, de manière davantage préférée, de plus de 500*500 pixels, de manière davantage préférée encore de plus de 1000*1000 pixels, comme de plus de 2000*2000 pixels, par exemple, de plus de 4000*4000 pixels, comme de plus de 8000*8000 pixels, par exemple, de plus de 10 000*10 000 pixels, comme de plus de 25 000*25 000 pixels, par exemple, de plus de 50 000*50 000 pixels, comme de plus de 100 000*100 000 pixels, par exemple, de plus de 250 000*250 000 pixels, comme de plus de 500 000*500 000 pixels, par exemple, de plus de 1 000 000*1 000 000 pixels.

27. Scanner selon la revendication 26, dans lequel la taille de pixel est la plus petite disponible sur le marché, par exemple dans lequel une section transversale d'un pixel est inférieure à 100 µm, comme inférieure à 50 µm, par exemple, inférieure à 25 µm, comme inférieure à 20 µm, par exemple, inférieure à 15 µm, comme inférieure à 10 µm, par exemple, inférieure à 7,5 µm, comme inférieure à 5 µm, par exemple, inférieure à 2,5 µm, comme inférieure à 2 µm, par exemple, inférieure à 1,5 µm, comme inférieure à 1 µm, par exemple, inférieure à 0,5 µm, comme inférieure à 0,25 µm, par exemple, inférieure à 0,1 µm, comme inférieure à 0,01 µm.

28. Scanner selon l'une quelconque des revendications précédentes, dans lequel l'au moins une caméra est sensible aux couleurs.

29. Scanner selon l'une quelconque des revendications précédentes, comprenant en outre un filtre dans la trajectoire de la lumière entre la surface de l'objet et la caméra, conçu pour filtrer la lumière provenant d'autres sources de lumière que les sources de lumière individuelles du scanner.

30. Scanner selon la revendication 1, comprenant au moins 2 caméras, comme au moins 3 caméras, par exemple, au moins 4 caméras.

31. Procédé pour balayer des surfaces intérieures comprenant les étapes consistant à :
i) pénétrer dans un scanner en forme de sonde ayant un axe dans une cavité.
ii) créer et projeter une lumière structurée depuis un premier point sur la sonde en produisant un motif sur une surface intérieure d'un objet et sur un second point de la sonde, enregistrer des images en 2D du motif réfléchi depuis la surface intérieure, en réalisant ainsi un balayage de 360° autour de l'axe de la sonde,
iii) déterminer les coordonnées en 2D des images du motif,
iv) déterminer la position et l'orientation relatives du scanner au cours des balayages successifs avec au moins un capteur de position,
v) combiner une série d'images pour obtenir des coordonnées universelles réelles en 3D de la surface intérieure.

32. Procédé selon la revendication 31, dans lequel le scanner est conforme à la revendication 1.

33. Procédé selon l'une quelconque des revendications 31 à 32, comprenant en outre l'étalonnage du scanner consistant à
i) balayer un objet d'étalonnage tridimensionnel ayant au moins un plan de symétrie et dans lequel au moins une partie d'au moins une courbe caractéristique de l'objet en 3D de chaque partie symétrique est une courbe continue,
ii) déterminer les coordonnées de caractéristiques d'image en utilisant des représentations d'au moins une paire de courbes de caractéristiques d'objet en 3D pour chacun d'un nombre discret de valeurs d'un angle de rotation et/ou d'une translation, d'une paire constituée d'une courbe caractéristique d'un objet en 3D dans chaque partie symétrique de l'objet d'étalonnage,
iii) changer les paramètres d'étalonnage pour s'adapter à l'objet d'étalonnage.

34. Procédé selon la revendication 33, dans lequel un motif lumineux est projeté sur l'objet d'étalonnage produisant des courbes de caractéristiques d'image en 2D.

35. Procédé selon la revendication 34, dans lequel les courbes de caractéristiques d'image en 2D sont déterminées au moyen de la transformée de Hough.

36. Procédé selon la revendication 33, dans lequel les coordonnées de caractéristiques d'image en 2D sont définies comme les intersections entre les courbes de caractéristiques d'image comme l'intersection entre deux courbes du n^{ième} ordre, comme l'intersection entre deux courbes du premier ordre, comme l'intersection entre deux courbes du deuxième ordre, comme l'intersection entre deux courbes du troisième ordre, comme l'intersection entre une courbe du premier ordre et une courbe du deuxième ordre, comme l'intersection entre une courbe du premier ordre et une courbe du troisième ordre ou comme l'intersection entre une courbe du deuxième ordre et une courbe du troisième ordre.

37. Procédé pour la modélisation et la production en 3D consistant à
obtenir des coordonnées universelles réelles en 3D d'une surface intérieure d'une cavité réalisée en utilisant le procédé selon l'une quelconque des revendications 31 à 33 et
créer une pièce conçue pour s'emboîter dans la cavité.

38. Procédé selon la revendication 37, dans lequel la production comprend le fraisage, l'impression tridimensionnelle, la stéréolithographie, le frittage laser sélectif, la modélisation d'objet laminé, la modélisation par jet d'encre, la modélisation par dépôt fondu, la nano-impression.

39. Procédé selon l'une quelconque des revendications 37 à 38, dans lequel la modélisation en 3D comprend la modélisation assistée par ordinateur des données de balayage avant production.

40. Procédé selon l'une quelconque des revendications 37 à 39, comprenant le balayage de la surface intérieure d'un canal auditif et produisant une coque d'aide auditive conçue pour loger un dispositif.
